Europäisches Patentamt

European Patent Office

Office européen des brevets

(11) Publication number: **0 216 310**
**B1**

(12) **EUROPEAN PATENT SPECIFICATION**

(45) Date of publication of patent specification: **06.12.89**

(51) Int. Cl.⁴: **A 61 K 35/78**

(21) Application number: **86112871.8**

(22) Date of filing: **18.09.86**

(54) **Production process of physiologically active substance from aloe.**

(30) Priority: **27.09.85 JP 214313/85**

(43) Date of publication of application:
**01.04.87 Bulletin 87/14**

(45) Publication of the grant of the patent:
**06.12.89 Bulletin 89/49**

(84) Designated Contracting States:
**DE FR IT**

(56) References cited:
**PATENT ABSTRACTS OF JAPAN, vol. 5, no. 200
(C-84)872r, 18th December 1981; & JP-A-56 120
621 (LION K.K.) 22-09-1981**

**PATENT ABSTRACTS OF JAPAN, vol. 6, no. 3
(C-86)881r, 9th January 1982; & JP-A-56 127
066 (LION K.K.) 05-10-1981**

**PATENT ABSTRACTS OF JAPAN, vol. 7, no. 86
(C-161)1231r, 9th April 1983; & JP-A-58 15 918
(RAION K.K.) 29-01-1983**

(73) Proprietor: **LION CORPORATION
3-7, Honjo 1-chome
Sumida-ku Tokyo (JP)**

(72) Inventor: **Hayashi, Tatsuo
14-12-163, Oogi-cho 5-chome
Odawara-shi Kanagawa (JP)**
Inventor: **Kameyama, Syoji
Sanitaun 211 471-2, Kuno
Odawara-shi Kanagawa (JP)**
Inventor: **Saito, Takeshi
2-11-201, Ayamedai
Chiba-shi Chiba (JP)**

(74) Representative: **Henkel, Feiler, Hänzel & Partner
Möhlstrasse 37
D-8000 München 80 (DE)**

Courier Press, Leamington Spa, England.

**Description**

The present invention relates to a process for producing a physiologically active substance having, for example, a wound curative activity, from Aloe.

It has been widely known in the art that an effective component having a wound curative activity is contained in Aloe such as *Aloe arborescens* Mill. var. *natalensis* Berger, and *Aloe vera*. L. Accordingly, various attempts have been made to fractionate an effective component having a wound curative activity from Aloe. The applicant proposed that a physiologically active substance be extracted from Aloe with a water-soluble organic solvent, followed by subjecting the substance to column chromatography, in Japanese Unexamined Patent Publication (Kokai) No. 56-120621, Japanese Unexamined Patent Publication (Kokai) 56-114301, and in Japanese Unexamined Patent Publication (Kokai) No. 58-15918, that a physiologically active substance be prepared by batchwise treating Aloe juice with activated carbon.

However, the former methods disclosed in Japanese Unexamined Patent Publication (Kokai) No. 56-120621 is disadvantageous from the practical point of view in that a large amount of water-soluble solvents such as alcohols, and an activated carbon column chromatography in which a large amount of activated carbon is used, are adopted, and the yield is not necessarily high. Thus, a relatively high cost is required to produce the desired physiologically active substance. In addition, the physiologically active substance obtained by this method has problems in the application thereof because it contains a relatively large amount of inorganic components and pain might be caused by the usage hereof. There are further problems in that the physiologically active substance is liable to be contaminated with water-insoluble components.

On the other hand, the above-mentioned problems can be solved by the latter method disclosed in Japanese Unexamined Patent Publication (Kokai) No. 58-15918. However, this method is disadvantageous in that, when the desired physiologically active substance is practically produced from Aloe, a large amount, i.e., 10% by weight or more, based on the Aloe, of activated carbon must be used to obtain a physiologically active substance having no substantial inflammatory properties and an excellent stability with the lapse of time. Accordingly, the cost is inevitably increased and various impurities included in the activated carbon are likely to be dissolved in and thus contaminate the desired final product. There are further problems in this method in that the separation of activated carbon is difficult from the viewpoint of practical operation, since the activated carbon is used in a relatively large proportion based on the liquid amount. Kokai No. 56-114301 refers to a simple purification with activated charcoal and does not describe the stepwise purification and concentration as described in the present invention.

Accordingly, the objects of the present invention are to eliminate the above-mentioned disadvantages of the prior art and to provide a process for producing, from Aloe, a physiologically active substance having an excellent physiologically activity such as an excellent wound curative activity, no substantial inflammatory properties, good application feeling, and a good stability.

Another object of the present invention is to provide a process for economically producing, from Aloe, the desired physiologically active substance containing a relatively small amount of activated carbon and having a good operability.

In accordance with the present invention, there is provided a process for producing a substance having a physiological activity, derived from Aloe comprising batchwise treating Aloe juice with activated carbon, optionally in combination with heat treatment, and recovering the liquid containing said substance by removing said activated carbon, characterized by comprising the steps of:

(i) batchwise treating Aloe juice with activated carbon as a first activated carbon treatment;

(ii) concentrating the treated juice after removing the activated carbon therefrom to a degree of concentration of the concentrate of from 5% to 20% by weight in terms of a solid content according to a Brix refractometer;

(iii) batchwise treating the liquid concentrate again with activated carbon as a second activated carbon treatment; and

(iv) recovering the treated liquid concentrate after removing the activated carbon therefrom.

Other objects and advantages of the present invention will be apparent from the following description.

According to the present invention, the desired physiologically active substance having stable qualities and derived from Aloe can be prepared by dividing the activated carbon treatment of Aloe juice into two separate batch operations, and prior to the second activated carbon treatment, concentrating the Aloe juice solution obtained by the first activated carbon treatment. As a result, the desired physiologically active substance having superior qualities can be obtained by decreasing the total amounts of the activated carbon used in the above treatments.

The present invention will now be explained in detail.

In the practice of the production of a physiologically active substance from Aloe according to the present invention, Aloe juice is first subjected to the first batchwise treatment with activated carbon. The starting Aloe materials usable in the present invention preferably include *Aloe arborescens* Mill. var. *natalensis* Berger, *Aloe vera*. L., and *Aloe saponaria* Haw. The Aloe juice can be obtained by, for example, squeezing, shredding, and/or grinding the total grass stems, the leaves, or the jellies obtained by removing leaf skins from the leaves. According to the present invention, the juice obtained by, for example, squeezing, shredding, and/or grinding the total grass stems, leaves, or jellies of Aloe is used as the starting material as mentioned above. As the starting material, the juice obtained by the above squeezing, shredding and/or grinding can be directly used, without removing solid materials contained in the juice, in the first activated carbon treatment. Preferably, however, from the operational viewpoint, the solid materials contained in the juice are removed by an appropriate separation means such as centrifugal separation, filter press separation, spontaneous separation using, for example, a filter paper or a filter fabric, and the resultant juice containing substantially no solid materials is used in the subsequent steps.

According to the present invention, the Aloe juice obtained as described above is treated with activated carbon. The first activated carbon treatment is carried out batchwise by adding the activated carbon to the juice, followed by stirring the mixture. Under the preferable conditions, the first batchwise treatment of the Aloe juice with activated carbon is carried out by using 2% to 10%, more preferably 5% to 8% (i.e., "% by weight" herein), based on the weight of Aloe, of the activated carbon at a temperature of 5 to 40°C for 30 minutes to 3 hours, more preferably 60 to 90 minutes.

After treating the Aloe juice in the first activated carbon treatment, the activated carbon is removed from the treated juice by, for example, filtration. The resultant juice is then concentrated by means of heating and/or pressure reduction. The degree of concentration of the juice is such that the solid concentration of the concentrated juice according to a Brix refractometer is 5% to 20%, more preferably 8% to 12%. Thus, when the juice after the first activated carbon is concentrated to a solid content of 5% to 20% according to a Brix refractometer, the amount of activated carbon used in the second activated carbon treatment can be advantageously decreased and the operational efficiency can be increased because the total volume of the substance to be treated is decreased.

According to the preesnt invention, the concentrated juice obtained above is further subjected to a second batchwise treatment with activated carbon. Thus, by dividing the batchwise treatment of the Aloe juice with activated carbon into two batchwise operations, and by treating the concentrated Aloe juice with the second activated carbon treatment, the total amount of the activated carbon used can be remarkably decreased and the operability thereof in the production process can be increased. Furthermore, although the amount of the activated carbon is decreased, the desired physiologically active substance having an excellent physiological activity, application feeling, and stability can be advantageously obtained.

Although there are no critical limitations to the second activated carbon treatment, the second activated carbon treatment is preferably carried out by using 0.1% to 5%, more preferably 0.5% to 2%, of the activated carbon, based on the weight of Aloe, at a temperature of 5°C to 40°C for 30 minutes to 3 hours.

The total amount of the activated carbon used in the first and second activated carbon treatments is preferably 3% to 10%, more preferably 5% to 8%, based on the weight of the Aloe.

After treating the Aloe juice in the second activated carbon treatment, the activated carbon is separated and removed from the treated Aloe juice by, for example, filtration, to obtain the desired crystal clear solution. The solution thus obtained can be directly used as a physiologically active substance. Alternatively, the resultant juice can be concentrated and dried to obtain a water-soluble stable white powder, which can be used as a physiologically active substance.

In the production process of the physiologically active substance according to the present invention, the Aloe juice is preferably subjected to a heat treatment prior to the first activated carbon treatment. The heat treatment is preferably carried out under an ambient pressure or a reduced or vacuum pressure at a temperature of 60°C to 95°C, especially 80°C to 95°C, for 20 seconds to 1 hour, especially 10 to 30 minutes. This heat treatment ensures the removal of undesirable water-insoluble components in the subsequent activated carbon treatment. According to this heat treatment, those components which are modified to water-insoluble components upon heat modification or with the lapse of time, or those components which will cause unpreferable discoloration or color change also can be completely removed. When the Aloe juice is heat treated at a temperature of less than 60°C or for less than 20 seconds, the above-mentioned advantageous effects to be obtained by the heat treatment may not be sufficient. Contrary to this, when the heat treatment temperature is higher than 95°C or the heat treatment time is more than 3 hours, the effective components might be modified.

The substance obtained according to the present invention has an extremely high curing acceleration activity against wounds such as cuts and burns and an excellent tissue activating action. Furthermore, the physiologically active substance thus obtained has a high stability, causes no substantial irritation when applied to the skin, and has a high stability, and therefore, can be suitably used, for example, in cosmetics and medicines for external application. In addition, since the physiologically active substance according to the present invention contains, for example, sugars, organic acids, amino acids, and water-soluble inorganic salts, has no toxicity when drunk and has a slightly sour taste, the physiologically active substance can also be usefully incorporated into food and drinks such as health drinks, and in oral products such as dentifrices, gargles, and troches.

As mentioned above, according to the present invention, the desired physiologically active substance having an excellent physiological activity such as a wound curative activity, and an excellent application feeling and stability, can be economically produced by dividing the activated carbon treatment of the Aloe juice into two separate batch operations and by concentrating the Aloe juice solution obtained by the first activated carbon treatment. Thus, the amount of activated carbon used in the treatments can be decreased and, therefore, the operability can be improved and the production cost decreased. Consequently, the present process is very suitable for use as an industrial production process.

# EP 0 216 310 B1

## Examples

The present invention will now be further illustrated by, but is by no means limited to, the following Examples.

## Example 1

A 10 kg amount of leaves of *Aloe arborescens* Mill. var *natalensis* Berger was shredded in a mixer and the pulp content was removed by filtration with a filter fabric. The resultant juice was heated at a temperature of 90°C for 20 minutes. After cooling, 600 g of activated carbon powder (i.e., Shirasagi P. available from Takeda Yakuhin Kogyo K.K.) was added to the juice and the mixture was then stirred for 1 hour at room temperature.

Thereafter, the mixture was filtered to obtain 6.5 liters of the clear filtrate. The filtrate was then concentrated to a solid content of 10% according to a Brix refractometer at a temperature of 60°C under a reduced pressure with an aspirator. After the concentration, 100 g of the same activated carbon powder as used above was added to the resultant concentrate, followed by stirring for 30 minutes at room temperature. The mixture was then filtered and the filtrate lyophilized. Thus, 160 g of the desired physiologically active substance in the form of a white powder was obtained.

## Comparative Example 1

A 10 kg amount of leaves of *Aloe arborescens* Mill. var. *natalensis* Berger was shredded in a mixer and the pulp content was removed by filtration with a filter fabric. The resultant juice was lyophilized to obtain 280 g of yellow-green powder.

## Comparative Example 2

A 10 kg amount of leaves of *Aloe arborescens* Mill. var. *natalensis* Berger was shredded in a mixer and the pulp content was removed by filtration with a filter fabric. The resultant juice was heated for 20 minutes at a temperature of 90°C. After cooling, 700 g of the same activated carbon powder as used in Example 1 was added to the juice and the mixture was then stirred for 1 hour at room temperature.

Thereafter, the mixture was filtered to obtain 6.2 liters of the clear filtrate. The filtrate was then concentrated to a solid content of 10% according to a Brix refractometer at a temperature of 40°C under a reduced pressure with an aspirator. The concentrate was then lyophilized. Thus, 180 g of a pale yellow powder was obtained.

## Comparative Example 3

A 10 kg amount of leaves of *Aloe arborescens* Mill. var. *natalensis* Berger was shredded in a mixer and the pulp content was removed by filtration with a filter fabric. The resultant juice was heated at a temperature of 90°C for 20 minutes. After cooling, 1300 g of the same activated carbon powder as used in Example 1 was added to the juice and the mixture was then stirred for 1 hour at room temperature. Thereafter, the mixture was filtered to obtain 4.8 liters of the clear filtrate. The filtrate was then concentrated to a solid content of 10% according to a Brix refractometer at a temperature of 40°C under a reduced pressure with an aspirator. After concentrating, the concentrate was lyophilized. Thus, 110 g of a white powder was obtained.

## Example 2

A 10 kg amount of leaves of *Aloe vera* L. was mixed with 10 liters of water and was shredded in a mixer. The mixture was heated at a temperature of 90°C for 30 minutes. After cooling, the pulp content was removed by filtration with a filter fabric. The resultant dark brown juice was charged with 800 g of activated carbon powder and the mixture was then stirred at room temperature for one hour.

Thereafter, the mixture was filtered to obtain 18.0 liters of the viscous pale yellow filtrate. The filtrate was then concentrated to a solid content of 5% according to a Brix refractometer at a temperature of 60°C under a reduced pressure with an aspirator. After the concentration, 200 g of the same activated carbon powder as used above was added to the resultant concentrate, followed by stirring for one hour at room temperature. The mixture was then filtered and the filtrate lyophilized. Thus, 104 g of the desired physiologically active substance in the form of a white powder was obtained.

## Comparative Example 4

A 10 kg amount of leaves of *Aloe vera* L. was mixed with 10 liters of water and was shredded in a mixer. The mixture was heated at a temperature of 90°C for 30 minutes. After cooling, the pulp content was removed by filtration with a filter fabric. The resultant dark brown juice was charged with 1800 g of the same activated carbon powder used in Example 2 and the mixture was then stirred at room temperature for one hour.

Thereafter, the mixture was filtered to obtain 17.0 liters of the relatively viscous clear filtrate. The filtrate was then concentrated to a solid content of 5% according to a Brix refractometer at a temperature of 60°C under a reduced pressure with an aspirator. The concentrated mixture was then lyophilized. Thus, 88 g of the white powder was obtained.

4

The physiologically active substance obtained in Examples 1 and 2 and the products obtained in Comparative Examples 1 to 4 were evaluated in the following Experiments 1 to 3.

Experiment 1

The wound curative activity of the sample obtained in Example 1 was evaluated using Wister male rats (body weight of 125 g to 135 g). The rats were divided into groups with each group containing 7 rats. The back skin of each rat was clipped over a wide area while the rat was anesthetized with pentbarbital. The clipped back skin was then disinfected with alcohol, a sharp cut having a length of 4 cm was made along the back center line with a knife, and the cut was stitched at three points at 1 cm intervals by a Michel needle.

After cutting, 40 mg/kg of body weight of rat of the sample (Example 1) dissolved in physiological saline was subcutaneously applied once a day for 7 successive days. After 7 days, the rat was killed with chloroform. The wound skin portion was stripped and the subcutaneous tissue was removed therefrom. Three skin strips having a width of 1 cm across the cutting line were obtained from the wound skin. The tensile strength required for tearing the wound skin strip was determined by means of an Instron tensile tester (i.e., Model 1130 Universal Test Instrument). The average tensile strength of three measurements obtained was used as an index of the wound curing degree. The results are shown in Table 1.

As a control, 2 ml/kg of body weight of rat of physiological saline was applied, instead of the sample solution, in the same manner as mentioned above. The tensile strength was determined in the same manner as mentioned above. The results are shown in Table 1.

TABLE 1

| Sample | Tensile strength (g/cm)±SE[1] | Ratio (%) |
|---|---|---|
| Control | 479.1±26.9 | 100 |
| Example 1 | 692.9±35.0[***] | 144.6[2] |

[1]: Average value of tensile strength was used as indication for wound curative degree.
[2]: Ratio of tensile strength of sample to that of control when tensile strength of control is designated as 100%.
[***]: $P < 0.001$.

As is clear from the results shown in Table 1, the physiologically active substance obtained in Example 1 according to the present invention exhibited a remarkable wound curative activity.

Experiment 2

The inflammatory activities of the samples obtained in Example 1 and Comparative Examples 1 to 3 were evaluated by using Slc:ddY male mice (age 6 weeks, 10 mice in each group). Each sample of Example 1 and Comparative Examples 1 to 3 was subcutaneously injected to the paw in an amount of 0.01 ml of 4% physiological saline solution. As a control, 0.01 ml of physiological saline was similarly applied to the opposite paw. After 5 hours from the time of application, both the paws were cut from the wrists, and the weights of the left and right paws were then measured.

The inflammatory activity was evaluated according to the increase in the ratio of the paw weight caused by the application of the sample to that of the control as follows.

Increased ratio of paw weight relative to control (%)

$$= \frac{A-B}{B} \times 100$$

A: weight applied by sample
B: control paw weight.

The results are shown in Table 2.

TABLE 2

| Sample | Increased ratio of paw weight relative to control (%)±SE | Statistical significant difference to sample of Example 1 |
|---|---|---|
| Example 1 | 0.73%±0.20 | — |
| Comparative Example 1 | 3.23%±0.27 | *** |
| Comparative Example 2 | 1.39%±0.18 | * |
| Comparative Example 3 | 0.69%±0.12 | N.S.[Note] |

*: $P<0.05$
***: $P<0.001$
[Note]: Non significant.

As is clear from the results shown in Table 2, the physiologically active substance of Example 1 according to the present invention exhibited no substantial inflammatory action. Contrary to this, the sample of Comparative Example 1, in which the activated carbon treatment was carried out once by using the same amount of the carbon as in Example 1, exhibited an inflammatory activity.

Furthermore, in the sample of Comparative Example 2, the activated carbon treatment was carried out once by using the total amount of the activated carbon used in the first and second activated carbon treatments.

As shown in Comparative Example 3, about twice the amount of the activated carbon based on the amount thereof in Example 1 according to the present invention must be used to obtan the product exhibiting no substantial inflammatory activity as in the physiologically active substance of Example 1.

Experiment 3

The stability with the lapse of time of the products of Examples 1 and 2 and Comparative Examples 1 to 4 was evaluated as follows.

A 20% aqueous solution of each product was prepared and allowed to stand for 3 hours in an oil bath at a temperature of 90°C. The absorbance at a wavelength of 420 nm was determined with the lapse of time. The results are shown in Table 3.

TABLE 3

| Example | Start | After 3 hours |
|---|---|---|
| Example 1 | 0.02 | 0.08 |
| Comparative Example 1 | 0.52 | 1.50 |
| Comparative Example 2 | 0.20 | 0.52 |
| Comparative Example 3 | 0.02 | 0.07 |
| Example 2 | 0.06 | 0.12 |
| Comparative Example 4 | 0.10 | 0.18 |

As is clear from the results shown in Table 3, the physiologically active substances of Examples 1 and 2 according to the present invention exhibited an excellent stability with the lapse of time. Contrary to this, when the Aloe juice was subjected to the activated carbon treatment only once, the amount of the activated carbon required for obtaining the stable product as in the present invention must be about twice that used in the Examples according to the present invention.

6

## EP 0 216 310 B1

### Claims

1. A process for producing a substance having a physiological activity and derived from Aloe comprising batchwise treating Aloe juice with activated carbon, optionally in combination with heat treatment, and recovering the liquid containing said substance by removing said activated carbon, characterized by comprising the steps of:

(i) batchwise treating Aloe juice with activated carbon as a first activated carbon treatment;

(ii) concentrating the treated juice after removing the activated carbon therefrom to a degree of concentration of the concentrate of from 5% to 20% by weight in terms of a solid content according to a Brix refractometer;

(iii) batchwise treating the liquid concentrate again with activated carbon as a second activated carbon treatment; and

(iv) recovering the treated liquid concentrate after removing the activated carbon therefrom.

2. A process as claimed in claim 1, wherein said Aloe is *Aloe arborescens* Mill. var. *natalensis* Berger or *Aloe vera* L.

3. A process as claimed in claim 1 or 2, wherein the Aloe juice is thermally treated prior to the first activated carbon treatment thereof under the conditions of a heating temperature of 60°C to 95°C and a thermal treatment time of 20 seconds to 1 hour.

4. A process as claimed in any one of claims 1 to 3, wherein the amounts of activated carbon are 2% to 10% by weight, based on the weight of the Aloe, in the first activated carbon treatment and 0.1% to 5% by weight, based on the weight of the Aloe, in the second activated carbon treatment.

5. A process as claimed in any one of claims 1 to 4, wherein the activated carbon treatment is carried out for 30 minutes to 3 hours in the first activated carbon treatment and for 30 minutes to 3 hours in the second activated carbon treatment.

### Patentansprüche

1. Verfahren zur Herstellung einer aus Aloe stammenden, physiologisch aktiven Substanz durch chargenweises Behandeln von Aloe-Saft mit Aktivkohle, gegebenenfalls in Kombination mit einer Wärmebehandlung, und Gewinnen der die betreffende Substanz enthaltenden Flüssigkiet durch Entfernen der Aktivkohle, gekennzeichnet durch folgende Stufen:

(1) chargenweises Behandeln von Aloe-Saft mit Aktivkohle als erste Aktivkohlenbehandlung;

(2) Konzentrieren des behandelten Safts nach Entfernen der Aktivkohle bis zu einem mittels eines Brix-Refraktometers ermittelten Konzentrationsgrad des Konzentrats von 5 bis 20 Gew.-% Feststoffgehalt;

(3) erneutes chargenweises Behandeln des flüssigen Konzentrats mit Aktivkohle als zweite Aktivkohlebehandlung und

(4) Gewinnen des behandelten flüssigen Konzentrats nach Entfernen der Aktivkohle aus diesem.

2. Verfahren nach Anspruch 1, dadurch gekennzeichnet, daß es sich bei der Aloe um Aloe arborescens Mill. var. natalensis Berger oder Aloe vera L. handelt.

3. Verfahren nach Ansprüchen 1 oder 2, dadurch gekennzeichnet, daß der Aloe-Saft vor seiner ersten Aktivkohlebehandlung während 20 s bis 1 h bei 60°C bis 95°C einer Wärmebehandlung unterworfen wird.

4. Verfahren nach einem der Ansprüche 1 bis 3, dadurch gekennzeichnet, daß, jeweils auf das Aloe-Gewicht bezogen, die Aktivkohlemengen bei der ersten Aktivkohlebehandlung 2—10 Gew.-% und bei der zweiten Aktivkohlebehandlung 0,1—5 Gew.-% betragen.

5. Verfahren nach einem der Ansprüche 1 bis 4, dadurch gekennzeichnet, daß die erste Aktivkohlebehandlung 30 min bis 3 h und die zweite Aktivkohlebehandlung 30 min bis 3 h durchgeführt werden.

### Revendications

1. Procédé pour la production d'une substance possédant une activité physiologique et extraite de l'aloès, comprenant un traitement par portions du suc d'aloès par le charbon actif, éventuellement en combinaison avec un traitement thermique et récupération du liquide contenant ladite substance en éliminant ledit charbon actif, caractérisé en ce qu'il comprend les étapes suivantes:

(i) traitement par portions de suc d'aloès par le charbon actif en tant que premier traitement par le charbon actif;

(ii) concentration du suc traité après en avoir éliminé le charbon actif à un degré de concentration du concentré compris entre 5% et 20% en poids en fonction d'une teneur en solides selon un réfractomètre Brix;

(iii) traitement par portions du concentré liquide encore une fois par le charbon actif en tant que deuxième traitement par le charbon actif; et

(iv) récupération du concentré liquide traité après en avoir éliminé le charbon actif.

2. Procédé selon la revendication 1, dans lequel ledit aloès est *Aloe arborescens* Mill. var. *natalensis* Berger ou *Aloe vera* L.

3. Procédé selon la revendication 1 ou 2, dans lequel le suc d'aloès est traité thermiquement avant le

7

premier traitement par le charbon actif dans les conditions d'une température de chauffage comprise entre 60°C et 95°C et une durée de traitement thermique comprise entre 20 secondes et 1 heure.

4. Procédé selon l'une quelconque des revendications 1 à 3, dans lequel les quantités de charbon actif sont comprises entre 2% et 10% en poids, basés sur le poids de l'aloès, dans le premier traitement par le charbon actif, et entre 0,1% et 5% en poids, basés sur le poids de l'aloès, dans le deuxième traitement par le charbon actif.

5. Procédé selon l'une quelconque des revendications 1 à 4, dans lequel le traitement par le charbon actif est effectué pendant une durée comprise entre 30 minutes et 3 heures dans le premier traitement par le charbon actif et entre 30 minutes et 3 heures dans le deuxième traitement par le charbon actif.